# EUROPEAN PATENT APPLICATION

(11) **EP 2 727 526 A1**
(43) Date of publication of application: **07.05.2014**
(21) Application number: 13191053.1
(22) Date of filing: 31.10.2013
(51) Int. Cl.: A61B 5/0402, A61B 5/02, A61B 5/0404, A61B 5/0205, A61B 5/024

(54) **Enhanced monitoring system, measurement apparatus and monitoring method**

(30) Priority: 31.10.2012 FI 20126133
(71) Applicant: Omegawave Oy, 02150 Espoo (FI)
(72) Inventor: Pinomaa, Juha, 02150 Espoo (FI)
(74) Representative: Boco IP Oy Ab

(57) **Abstract**

A system in which two or more different measurement processing procedures are included in a personal unit attached to the user. These procedures are applicable for computing measurement data from a single signal generated by a sensory system of the personal unit. The measurement processing procedure to be presently applied is selected according to control information received from a separate monitoring unit. The present invention provides a simple way to provide more versatile measurement results and make them accessible to a variety of different user applications.

## Description

### Field of the invention

The present invention relates to physiological state evaluations, and especially to a system, an apparatus, a method, and a computer program product for generating values applicable in evaluations of a physiological state of a user.

### Background of the invention

With the recent progress of sensor techniques and information processing, non-invasive measurements and physiological state monitoring systems have quickly evolved to a range of non-medical uses. Athletes wish to accurately monitor progress of their training on a frequent basis, and even ordinarily training people are interested to follow their effort and effects of it.

However, present monitoring systems are still quite simple and use measured information in a very limited manner. This is mainly due to limitations in generation of the measurement values. Traditional monitoring systems were designed to patients that typically do not move, and do not mind having a number of different sensors and sensor systems attached to them during the measurement. The situation is, however, completely different in the non-medical side, where measured values need to be generated with minimal disturbance to the monitored person and with least effort. For measurements made in normal training conditions, people do not wish to purchase several different sensor systems, attach them to their bodies, and try to make sure that sensors remain appropriately connected also during training.

For example, there are athletes and other trainers that may wish to use an advanced monitoring system that applies both measured electrocardiography (ECG) and measured heart rate variability (HRV) values to generate a comprehensive visual output on their current physiological state. These measurements may be needed at different times, for example, the athlete may wish to make a holistic assessment using measured ECG values in the morning and apply measured HRV values during a workout. Presently, in order to be able to apply both of these measurement types, the athlete needs to purchase two separate sensory systems for each of these measurement types, and input outputs of these sensory system manually to the monitoring system. As can be understood, not many users are willing to establish such systems and continuously go through the laborious manual input steps.

Some advanced commercial sports training computers and heart rate monitoring equipment may already be integrated to a monitoring system where measured values may be automatically processed, and combined to user input profile information and to earlier measured information on the user. However, the attached user equipment is typically only capable to transmit only one type of signal, and apply it only in dedicated personal monitoring devices and applications. The signal may comprise one or two types of values characteristic of the functions of the heart, but the user can only apply only features and functions made available by the monitoring applications of the equipment vendor. Whenever one wishes to acquire values computed in a different manner from the ECG sensor signals, or values generated by personal user equipment in a different format, one needs to purchase new user equipment and/or laboriously manage the integration of the measurement results.

### Brief description of the invention

The object of the present invention is to provide a system, a method, an apparatus and a computer program product so as to overcome at least some of the prior art disadvantages. The objects of the present invention are achieved with a system according to the characterizing portion of claim 1. The objects of the present invention are further achieved with an apparatus according to the characterizing portion of claim 2, a method according to the characterizing portion of claim 6, a monitoring apparatus according to the characterizing portion of claim 10 and with a computer program product according to the characterizing portion of claim 11.

The preferred embodiments of the invention are disclosed in the dependent claims.

The present invention is based on the idea of including in a personal unit attached to the user two or more different measurement processing procedures, which are applicable for computing measurement data from a signal generated by a sensory system of the personal unit. The measurement processing procedure to be presently applied is selected according to control information received from a separate monitoring unit.

The present invention provides a simple way to provide more versatile measurement results and make them accessible to a variety of different user applications. Further advantages of the invention will be discussed with descriptions of respective embodiments of the invention.

### Brief description of the figures

In the following the invention will be described in greater detail, in connection with preferred embodiments, with reference to the attached drawings, in which
Figure 1 illustrates basic elements of an embodiment of a system according to the present invention;
Figure 2 illustrates functional elements of a measurement module in a personal unit;
Figure 3 illustrates steps of an embodiment of a method implemented in the monitoring unit;
Figure 4 illustrates steps of an embodiment of a method implemented in the personal unit;
Figure 5 illustrates an alternative element configuration for the logical system structure of Figure 1;
Figure 6 illustrates configuration of an exemplary apparatus applicable as a system element for embodiments of in the present invention.

### Detailed description of some embodiments

The following embodiments are exemplary. Although the specification may refer to "an", "one", or "some" embodiment(s), this does not necessarily mean that each such reference is to the same embodiment(s), or that the feature only applies to a single embodiment. Single features of different embodiments may be combined to provide further embodiments.

Features of the invention will be described with simple examples of a system architecture in which various embodiments of the invention may be implemented. Only elements relevant for illustrating the embodiments are described in detail. Various implementations of the invented methods and devices comprise elements that are generally known to a person skilled in the art and may not be specifically described herein.

The invention relates to generation of values that represent a physiological state of a person. The physiological state of a person is a broad concept and may be monitored and estimated by means of several different physiological functions. Various types of physiological parameters may be applied, alone or in combination, to create an output that is comprehensive and indicative of the physiological state of the person. One important and very frequently monitored object is the heart, the activity of which may be non-invasively detected with sensor systems that are attached to the skin, and recorded with a monitoring unit that is external to the body of the user.

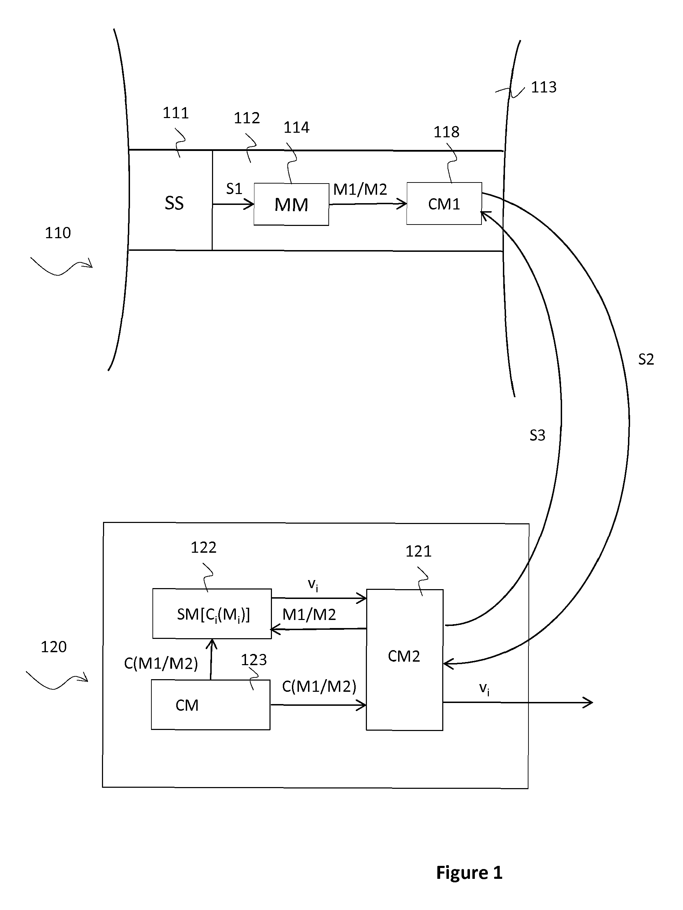

Figure 1 illustrates basic elements of an embodiment of a system according to the present invention. The system comprises a personal unit 110 and a monitoring unit 120. The personal unit 110 and the monitoring unit 120 are communicatively connected to each other and can therefore exchange information.

The personal unit 110 comprises a sensory system 111 and a device 112 for detachably attaching the sensory system 111 to the outer surface of a skin of a user 114. A sensor in general refers to a device that responds to a physical stimulus, and upon detecting such stimulus, transmits a corresponding impulse. The term sensory system herein may comprise a combination of one or more sensors that are responsive to electrical potentials generated on the skin when the heart muscle depolarizes during heartbeats. The one or more sensors may be positioned into contact with the outer surface of a skin of a user, and generate an electrical signal in response to detected electrical potentials. Signals of the sensors of the sensory system are integrated to generate a first signal S1, a common output signal that varies according to electric activity of the myocardium.

The device 112 refers here to mechanical means that may be applied to position the sensors of the sensory system 111 into contact with the outer surface of the skin of a user 113. The device 112 may be implemented, for example, with a strap, at least part of which is elastic and which has a releasable locking mechanism. The sensors and any electrical wiring required by their electrical connections may be attached or integrated to one surface of at least part of the strap. When the releasable lock is closed, this one surface of the strap may be positioned against the skin of the user and due to the stretching of the elastic part of the strap, the sensors press against the skin such that appropriate electrical contact for detecting the electrical potentials on the skin is achieved. The strap may be formed as a separate belt fastened around the chest of the user, but other forms for the device may be applied, as well. For example, the device 112 may be formed as a piece of garment, for example as a sports top or fitted shirt with smart conductive fabric or sensors incorporated into the fabric. In addition to elasticity, the device may apply other means of attachment, as well. For example, the device 112 may comprise easily removable adhesive bands between the sensors and the skin for the purpose.

The personal unit 110 comprises also a measurement module 114 that is electrically connected to the sensory system 111. The measurement module 114 receives the first signal S1 from the sensory system 111, and processes it into measurement data applicable in the monitoring unit.

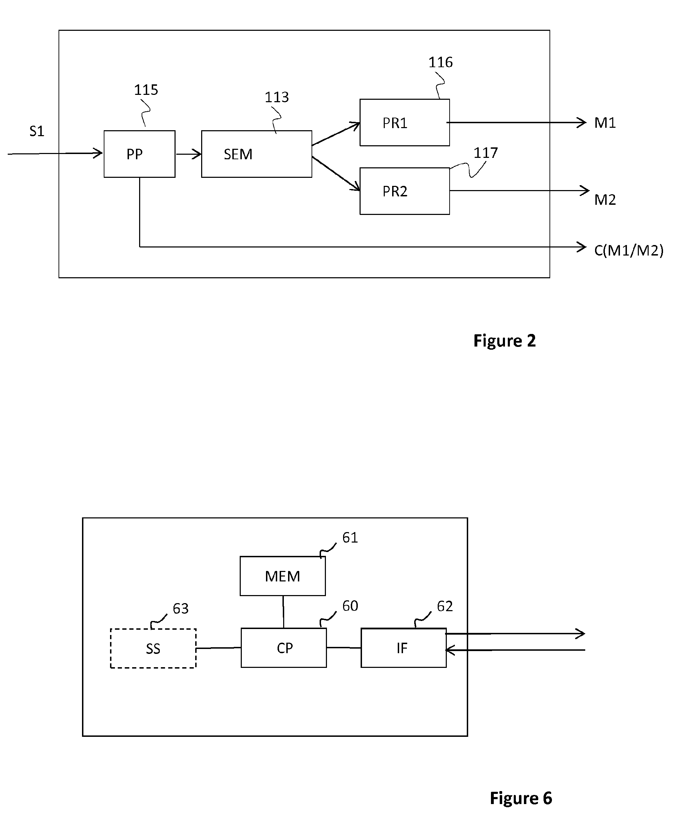

The block chart of figure 2 illustrates in more detail functional elements of the measurement module 114. Typically the first signal S1 from the sensory system is an analog sensor signal. In order to allow digital computing, the measurement module 114 may comprise a preprocessing module 115 with a preamplifier that amplifies the sensor signal S1, and an analog-to-digital converter to convert the analog sensor signal S1 to a digital form signal S1*. Other necessary signal processing methods may be applied at this stage, as well. Such conventional signal processing functions are well known to a person skilled in the art and will not be separately described in more detail herein.

In the present embodiment, the measurement module 114 may comprise also at least two different measurement processing procedures 116, 117 for computing the measurement data from the first sensor signal S1/S1*. A measurement processing procedure may incorporate one or more software analysis and conversion tools that convert the raw data received from the sensory system into measurement data.

As an example, let us assume that the personal unit comprises two measurement processing procedures. The first measurement processing procedure could comprise an algorithm that uses information in the first signal to compute values modeling current ECG values of the user. In medical use, ECGs are usually performed for diagnosis of heart abnormalities. For required medical accuracy, a full 12-lead diagnostic ECG applies ten electrodes attached to predefined locations on the body of the patient. The term lead refers here to a separate electrical connection between an electrode and an ECG recorder. In limited clinical and research applications, already a 6-lead ECG with 5 electrodes may provide acceptable accuracy. In non-medical use, an ECG system with less than 6 leads, two at minimum, may be applied to model ECG values of the person. In the present example, the analog signal from the two electrodes may sampled (e.g. sample rate 500 Hz) and processed to digital form S1*. For improved accuracy, the analog signal S1 may be oversampled (for example, 16x500Hz). The first measurement processing procedure may thus comprise an algorithm that inputs samples of the analog signal generated in the preprocessing module and outputs modeled ECG data M1 in digital form from the measurement module.

The second measurement processing procedure could comprise an algorithm that uses information in the first signal to compute values that indicate the present heart rate variability (HRV) of the user. Heart rate variability (HRV) is a physiological phenomenon of variation in the time interval between heartbeats. It is measured by the variation in the beat-to-beat interval, often determined from a time interval between predefined characteristic points (R-points) of an ECG curve. The second measurement processing procedure may thus comprise an algorithm that inputs samples of the analog signal generated in the preprocessing module and computes from them time intervals between R points and outputs these successive time intervals M2 from the measurement module. These successive interval durations may be used in the monitoring unit to the variations in the beat-to-beat intervals. Alternatively, algorithm of the second measurement processing procedure may be configured to compare the value of the present interval to a predefined reference value and output the measured value as difference to the predefined reference value.

Either of the resulting measurement data M1, M2 carries meaningful information for a specific monitored characteristic in the operation of the heart, and is structured to a defined data format used in an application of the monitoring unit. The two or more procedures 116, 117 of the measurement module 114 are different from each other. This means that computation with values of one signal S1/S1* with one procedure PR1 116 provides a measurement data set M1, and computation with the same values with another procedure PR2 117 provides a measurement data set M2, and M1 and M2 are different from each other. The procedures may be configured to output M1 with format that differs from M2. Alternatively, the algorithms used to analyze or model the changes in the electrical potential for M1 and M2 may be different, and therefore provide different resulting values in computation. The procedures may also be configured to both apply different algorithms and generate different formats for values of M1 and M2. The number of measurement processing procedures and measurement data types is not limited to two, the measurement module may comprise additional alternative processing paths, as well. For example, the measurement module may comprise a third measurement processing path for conventional heart rate measurements where the output simply indicates the number of heartbeats per unit of time. For conciseness, a configuration of two measurement processing procedures is described herein. Application of the description to configurations with more than two measurement data types is clear to a person skilled in the art.

As shown in Figure 1, the personal unit 110 comprises also a first communication module 118 configured to enable exchange of information between the personal unit 110 and the monitoring unit 120. Advantageously the personal unit 110 is a wearable element, so the first communication module 118 should be mechanically light and operate with minimal power consumption in the side of the personal unit 110. Typically the communication link between the personal unit 110 and the monitoring unit 120 is thus implemented with a simple non-networked connection with short range accessibility. Examples of wireless short range non-networked communication technologies comprise Bluetooth, IrDA, Wireless USB, Z-Wave, ZigBee, among others. Also wired computer buses, such as USB and FireWire, may be applied for the purpose. The first communication module 118 is configured to incorporate the measurement data M1 or M2 received from the measurement module into a second signal S2, and transmit it to the monitoring unit 120. The first communication module 118 is also configured to receive a third signal S3 that carries control data C(M1/M2) from the monitoring unit.

As shown in figure 2, the measurement module 114 of the present embodiment comprises a selection module 119, which receives the first signal and is configured to activate one measurement processing procedure from the group of at least two different measurement processing procedures for computing the measurement data. The selection module 119 is arranged to be responsive to the control data C(M1/M2) received from the monitoring unit. Accordingly, when control data C(M1/M2) is received from the monitoring unit 120 to the personal unit 110, the first communication module 118 forwards it to the selection module 119, and the selection module decides which measurement processing procedure 116, 117 to activate according to the received control data C(M1/M2).

As shown in Figure 1, the monitoring unit 120 comprises a second communication module 121 that is configured to receive from the personal unit 110 the second signal S2. The second signal S2 carries the measurement data M1/M2 generated with one of the activated procedures 116, 117 from the first signal S1. The monitoring unit 120 comprises also a state module 122 configured to compute from the received measurement data M1/M2 one or more output values that represent the physiological state of the user 113. The state module 122 comprises two or more different computing methods CP1, CP2 applicable for computing output values from the received measurement data. Each of the computing methods CP1, CP2 corresponds to one measurement processing procedure PR1, PR2 used for computing the measurement data M1, M2 in the personal unit 110. In order to ensure that measurement data is appropriately applied in the state module 122, the monitoring unit comprises a control module 123.

The control module 123 is configured to generate the control data C(M1/M2) with which the selection module 119 of the personal unit 114 may select a procedure 116, 117 to be used to compute the measurement data M1/M2. The control module 123 is configured to ensure synchronization of operations in the selection module 119 of the personal unit 110 and the state module 122 of the monitoring unit 120. For this, the control module 123 forwards control data C(M1/M2) for selection of a procedure PR1 for M1, or PR2 for M2 to the second communication module 121. At the same time the control module 123 activates in the state module 122 a computing method CP1 or CP2 that corresponds with the indicated measurement processing procedure PR1 or PR2, respectively, in the control data C(M1/M2).

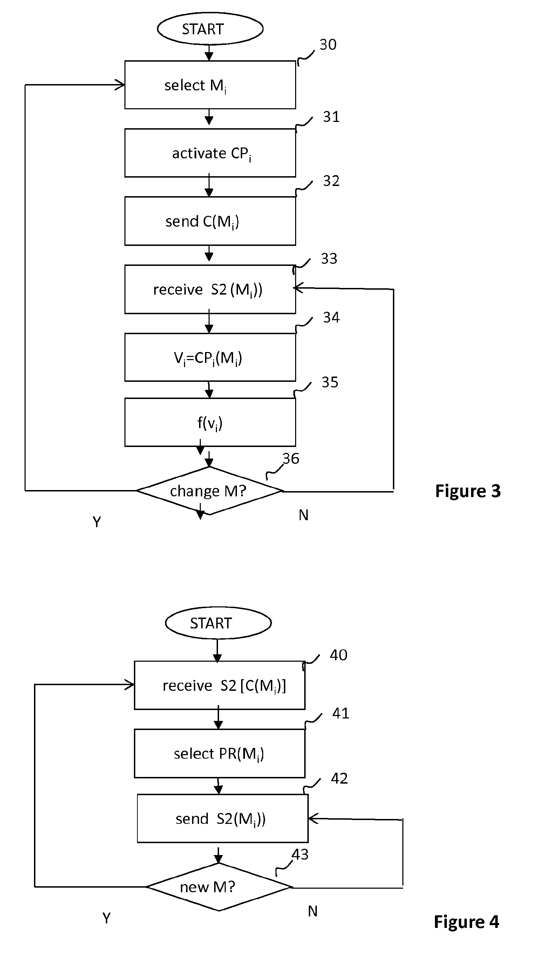

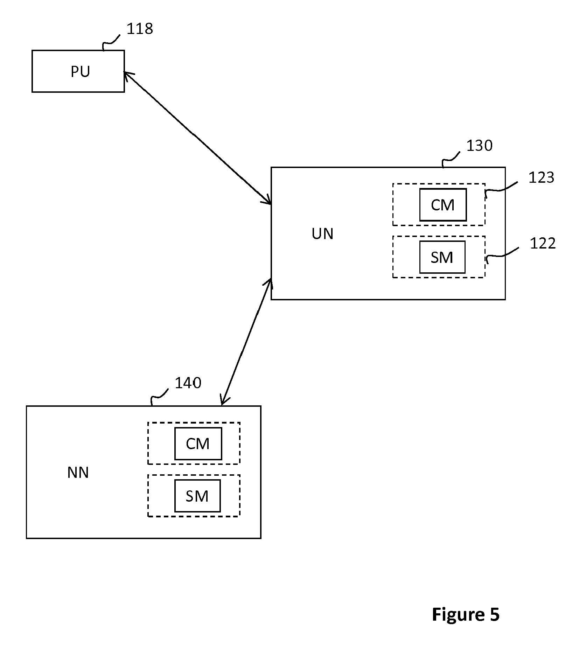

Operation of the system described in Figures 1 and 2 is illustrated with the flow chart of Figure 3. The flow chart shows steps of an embodiment of a method according to the present invention, implemented in the monitoring unit 120 of Figure 1. The procedure begins at a stage where both units 110, 120 have been initialized for use and in operative state. In the beginning, the control module determines what type of measurement data Mi is to be generated next (stage 30). As will be discussed in more detail with Figure 5, the control module may make the decision on various grounds, for example, based on user input, based on triggering command received from a physical state monitoring program, timed according to a predefined schedule, or as a combination of these.

When the measurement data type Mi has been selected, the control module activates (stage 31) in the state module a computing method CPi that corresponds to the measurement data type Mi and/or the defined measurement processing procedure PRi applied for computing the measurement data type Mi. The control module also creates control data C(Mi) that indicates the same measurement data type Mi and/or the defined personal unit procedure PRi applied for computing the measurement data type Mi, and forwards it to the second communication module for delivery in signal S3 to the personal unit (stage 33). Now when measurement data Mi of the selected type is received in S2 from the personal unit (stage 34), the state module is ready to apply it in the correct way and form. It is understood that delivery and processing of the control data to and from the personal unit evidently causes some delay before the desired type of measurement data begins to flow. If necessary, activation of the computing method may be synchronized to beginning of reception of desired type of information, for example, by some simple marker in the beginning of a data stream of a new type. Any other synchronization method may be applied, as well, and any other checking, verification, acknowledgement procedures or the like may be applied in the procedure without deviating from the scope of protection.

Accordingly, when the computing method CMi has been activated and measurement data Mi in the corresponding form is received, the state module may use the measurement data Mi to compute with it one or more values Vi that represent the physiological state of the user of the personal unit (stage 34). The monitoring unit may use these values in one or more monitoring functions assigned to it (stage 35). For example, the monitoring unit may comprise and maintain a physiological state monitoring program that collects various types of measurement and input data on a monitored person and processes them into visual presentation and makes this available to the user and people authorized by him/her. In such implementation, the function may comprise inputting the computed values Vi to the physiological state monitoring program for further use. The physiological state monitoring program may also be provided as a separate service in some other entity, and in such implementation, the function may comprise transmitting the computed values Vi to the entity operating the service. Other functions are possible as well, the invention is not, as such, limited to any specific purpose or use of the values.

In the exemplary embodiment of Figure 3 the change of measurement data type is explicit, meaning that the personal unit will continue providing a specific measurement data type until it has been explicitly changed with a command from the monitoring unit. The control unit will thus continuously check whether some other type of measurement data is needed or requested (stage 36). If this is the case, the process returns back to stage 30 to select the new type for the measurement data. Otherwise the monitoring unit continues receiving measurement data of the selected type in stage 32. As discussed above, the decision may be implicit such that transition from a selected measurement data type to a default data type occurs as timed from activation of the selection in the personal unit and activation of the corresponding computing method in the monitoring system.

The flow chart of Figure 4 shows steps of an embodiment of a method according to the present invention, implemented correspondingly in the personal unit 110 of Figure 1. The procedure begins at a stage where both units 110, 120 have been initialized for use and in operative state. In the beginning, the personal unit receives the signal S3 that includes the control data C(Mi) to indicate the measurement data type Mi and/or the defined measurement processing procedure PRi applied for computing the desired measurement data type Mi (stage 40). In response to this control information, the selection unit of the personal unit begins to channel information from the first signal S1 to the selected measurement processing procedure (stage 41). The measurement data Mi sent in the second signal S2 from the personal unit to the monitoring unit thus includes measurement data in the form decided by the monitoring unit. The personal unit continuously monitors whether change of measurement type is necessary (stage 43). If change is needed, the process returns to stage 40 for a new selection, otherwise it will return to stage 42 and continue sending the measurement data in the selected format.

As discussed earlier, the personal unit 110 is typically a lightweight personal element of the user. Elements of the monitoring system 120 may be divided to various physical elements, depending on the application. The configuration of Figure 1 is a logical presentation of functional elements required for operations of the monitoring unit, but it may also be considered as an illustration of a possible physical system structure where both the apparatus for measurement and the apparatus for monitoring are operated by the user.

Such configuration could be implemented with, for example, a combination of a wearable personal unit and a personal computing device, interconnected with a short range communication link, like Bluetooth.

Let us continue with the earlier practical example where the personal unit comprises two procedures, one for modeling ECG values and one for measuring HVR values of the user. In this exemplary embodiment, the personal computing device comprises a personal fitness monitoring application that applies both HVR values and ECG values, potentially in combination with a number of other input values. The application may be configured to use the personal unit to record and monitor ECG by default, but to allow the user to initiate a period during which a measurement for more comprehensive and holistic illustration of the present fitness state of the user is generated. Typically a user is only able to provide display control commands to predefined output windows of the application, but with the arrangement of the present invention, the user can actively control which physical functions are monitored, and when.

For example, in the present example, let us assume that the monitoring unit is provided by a personal computer device, like a mobile phone. During normal operation, the personal unit generates the first signal and processes with one computing procedure modeled ECG values that it delivers to a personal fitness monitoring application in the monitoring unit, and the application displays these values through the user interface of the mobile phone. At some point, decided by the user, he requests via the application user interface display more comprehensive monitoring information, for which also present HVR values are needed. In response to a user request, the application generates control data to change the type of measured data and delivers it to the personal unit. According to the invention, in response to the control data, the personal unit continues to use the same first signal generated by the same sensory system as with the ECG modeling, but processes the first signal differently so that HVR values are output. These values are transmitted to the monitoring unit and used in the application for the enhanced fitness monitoring display. Measurements can thus be controlled by the user and initiated and at any time, independently selected by the user.

In addition, with the present invention, the user may gain also gain even further flexibility to the monitored states. As discussed earlier, physiological state is a general concept and can be modeled from various aspects. Typically one application comprises only a limited set of monitored aspects, based on a predefined set of monitored parameters. The personal unit and the monitoring application have been configured to communicate via predefined protocols, and for further views, the whole set of equipment has been changed for new measurements, even if part or most of the information has been based on measurements made on the heart. In the present invention, the one and the same personal unit may be adapted to generate measurement data for two or more applications, i.e. to provide the type of measurements used by the different applications, and in the specific format of each of the applications. This is facilitated by the specific functional system structure, described with Figures 1 and 2.

Figure 5 illustrates an alternative element configuration for the logical system structure of Figure 1. The system of Figure 5 comprises the personal unit 110, but the monitoring unit 120 has now been divided into two physical system elements, a user node 130 and a network node 140. The user node and the network node are connected over a wide area network, which may include various types of well-known wireless and wired network links. Depending on application, the state module 122 and the control module 123 may be implemented in either or both of the nodes.

In one embodiment, both the state module and the control module may be implemented in the network node 140. The network node may be an application server that provides fitness monitoring application as a service to a plurality of users. In such configuration the user node 130 may be a personal computing device into which a terminal application part of the fitness monitoring application has been installed. The state module 122 and the control module 123 are operated in the application server as described in Figures 1 to 4, and the role of the user node is to relay the second signal S2 and the third signal S3 between the personal unit 110 and the application server 140. Specifically, the user node associates in a user-friendly way and in a verified manner the measured data with a correct user identity. Since most of the computing is done in the application server side, this configuration allows dynamic use of various powerful and continuously enhancing monitoring tools with minimal user equipment, and user participation. The application server may comprise a user interface for manual control operations of an external monitoring operator.

In another embodiment, the state module 122 may be in the network node 140 and the control module 123 in the user node 130. The user node may comprise a user interface through which the user may input commands to control the monitoring procedures and thereby select types of measurement data currently applied. For example, the user node 130 may comprise a terminal application with which the user may select to monitor continuously his ECG or heart rate, but at some point use the terminal application to trigger a mode during which the one and same sensor signal is applied to produce HVR for more holistic monitoring applications. Alternatively, the user node may comprise two or more terminal applications that the user may activate at selected times to collect and display different types of monitoring data. For example, in the present exemplary case, either of the terminal applications may use HRV and ECG type of measured data in connection with other type of measurement information. The terminal applications may be configured to generate control information in a format applicable by the selection module 119 of the measurement module 114. A much larger view to physiological state of the user is achieved in a simple manner; no new user equipment needs to be acquired and installed for the purpose.

In other embodiments, functions of the state module 122 and the control module 123 may be further divided to the user node 130 and the network node 140. Some of the control functions may be implemented as timed by a monitoring application, either triggered by the terminal application part or by the server application. Some of the control functions may be initiated by user commands, given by the monitored person via the terminal application, or by an external monitoring operator via the server application. Part of the computations of the state module may be performed already in the user node and delivered to the network node for storing or for further calculations. Further implementations of the division may be applied without deviating from the scope of protection.

Figures 1 and 2 illustrate an embodiment of the system with logical elements that represent functions implemented in the personal unit and monitoring units, and Figures 3 and 4 illustrate methods implemented in these units. Embodiments of this invention comprise also an apparatus, applicable as a personal unit or a monitoring unit according to the invention. The apparatus may be a computing device that provides means for implementing the functions and methods described with Figure 1 to 4. Figure 6 shows a block diagram illustrating configuration of an exemplary apparatus for the purpose. It is noted that any type of computing devices specifically devised and adapted to carry out the disclosed functions or methods may be applied without deviating from the scope of protection. Advantageously the computing device of the attached personal unit is a simple and lightweight device configuration with modest processing, memory and communication means. A user node as the monitoring device may provide more computing and communication resources and even more processing resources may be taken into use in an application server.

The apparatus comprises a processor unit 60 for performing systematic execution of operations upon predefined data. The processor unit 60 is an element that essentially comprises one or more arithmetic logic units, a number of special registers and control circuits. Memory unit 61 provides a data medium where computer-readable data or programs, or user data can be stored. The memory unit is connected to the processor unit 60. The memory unit 61 typically comprises volatile or non-volatile memory, for example EEPROM, ROM, PROM, RAM, DRAM, SRAM, firmware, programmable logic, etc. The apparatus also comprises an interface unit 62 with at least one input unit for inputting data to the internal processes of the apparatus and at least one output unit for outputting data from the internal processes of the apparatus.

If a line interface is applied, the interface unit 62 typically comprises plug-in units acting as a gateway for information delivered to its external connection points and for information fed to the lines connected to its external connection points. If a radio interface is applied, the interface unit 62 typically comprises a radio transceiver unit, which includes a transmitter and a receiver, and is also electrically connected to the processing unit 50. The transmitter of the radio transceiver unit receives a bitstream from the processing unit 60, and converts it to a radio signal for transmission by the antenna. Correspondingly, the radio signals received by the antenna are led to the receiver of the radio transceiver unit, which converts the radio signal into a bitstream that is forwarded for further processing to the processing unit 60. Different radio interfaces may be implemented with one radio transceiver unit, or separate radio transceiver units may be provided for the different radio interfaces.

The interface unit 62 of an apparatus, especially one used as a user node or a network node, may also comprise a user interface with a keypad, a touch screen, a microphone, and equals for inputting data and a screen, a touch screen, a loudspeaker, and equals for outputting data. An apparatus used as a personal unit may also comprise a sensory system 63.

The processor unit 60, the memory unit 61, and the interface unit 62 are electrically interconnected to provide means for performing systematic execution of operations on the received and/or stored data according to predefined, essentially programmed processes of the apparatus. These operations comprise the procedures described for the personal unit and the monitoring unit.

In general, various embodiments of the apparatus may be implemented in hardware or special purpose circuits, software, logic or any combination thereof. Some aspects may be implemented in hardware, while some other aspects may be implemented in firmware or software, which may be executed by a controller, microprocessor or other computing device. Software routines, which are also called as program products, are articles of manufacture and can be stored in any apparatus-readable data storage medium and they include program instructions to perform particular predefined tasks. The exemplary embodiments of this invention also provide a computer program product, readable by a computer and encoding instructions for executing a process for generating output values representing physiological state of a person in an apparatus of Figure 6.

While various aspects of the invention may be illustrated and described as block diagrams, message flow diagrams, flow charts and logic flow diagrams, or using some other pictorial representation, it is well understood that the illustrated units, blocks, apparatus, system elements, procedures and methods may be implemented in, for example, hardware, software, firmware, special purpose circuits or logic, a computing device or some combination thereof.

It is apparent to a person skilled in the art that as technology advances, the basic idea of the invention can be implemented in various ways. The invention and its embodiments are therefore not restricted to the above examples, but they may vary within the scope of the claims.

## Claims

1. A system, comprising a personal unit and a monitoring unit interconnected for communication;
the personal unit comprises:
a sensory system and a device for detachably attaching the sensory system to the outer surface of a skin of a user, the sensory system being configured to generate a first signal representing electrical changes on the outer surface of the skin of the user;
a measurement processing module connected to the sensory system and configured to process the first signal into measurement data, the measurement processing module comprising a group of at least two different measurement processing procedures for computing the measurement data from the first signal,
the monitoring unit comprises:
a state module configured to compute from the received measurement data one or more output values representing a physiological state of the user, the state module comprising two or more different computing methods for computing the one or more output values from the received measurement data, each of the computing methods corresponding to one measurement processing procedure of the personal unit;
a control module configured to provide to the personal unit control data for selecting a measurement processing procedure, and to activate a corresponding computing method in the state module;
wherein
the measurement processing module comprises a selection module, configured to activate one measurement processing procedure from the group of at least two different measurement processing procedures according to the control data received from the monitoring unit.

2. An apparatus comprising:
a sensory system and a device for detachably attaching the sensory system to the outer surface of a skin of a user, the sensory system being configured to generate a first signal representing electrical changes on the outer surface of the skin of the user;
a measurement processing module connected to the sensory system and configured to process the first signal into measurement data computed with a predetermined measurement processing procedure from the first signal;
a first communication module configured to send the measurement data to a monitoring unit; wherein
the measurement processing module comprises a group of at least two different measurement processing procedures for computing the measurement data from the first signal, and the measurement processing module comprises a selection module, responsive to control data received from the monitoring unit, and configured to activate one procedure from the group of measurement processing procedures according to the control data received from the monitoring unit.

3. An apparatus according to claim 2, **characterized in that** the sensory system comprises a combination of two or more electrodes that are responsive to electrical potentials generated on the skin of a user when the heart muscle of the user depolarizes during heartbeats, the two or more electrodes being combined to generate an analog first signal.

4. An apparatus according to claim 3, **characterized by** comprising a measurement processing procedure for outputting digital values modeling present ECG of the user and a different measurement processing procedure for outputting time interval values between successive characteristic points in the first signal.

5. An apparatus according to claim 4, **characterized by** comprising a third measurement processing procedure for outputting heart rate values indicating the number of heartbeats per unit of time.

6. A method, comprising:
selecting from a group of two or more different measurement processing procedures a measurement processing procedure to be used in computing measurement data from one sensor signal in a personal unit;
generating control data indicating the selected measurement processing procedure;
sending the control data to the personal unit;
activating from a group of two or more different computing methods a computing method corresponding to the selected measurement processing procedure;
computing with the activated computing method from the measurement data one or more output values representing a physiological state of the user.

7. A method of claim 6, wherein the steps of the method are performed in a user node or a network node of a monitoring system.

8. A method of claim 6, wherein all steps of the method are performed in the user node of the monitoring system.

9. A method of claim 6, wherein all steps of the method are performed in the network node of the monitoring system.

10. A monitoring apparatus, configured to execute the method of any of claims 6 to 9.

11. A computer program product, readable by a computer and encoding instructions for executing a method of any of claims 6 to 9 in a monitoring apparatus.
